(19) Europäisches Patentamt
European Patent Office
Office européen des brevets

(11) **EP 4 171 667 B1**

(12) **EUROPÄISCHE PATENTSCHRIFT**

(45) Veröffentlichungstag und Bekanntmachung des Hinweises auf die Patenterteilung:
**26.02.2025 Patentblatt 2025/09**

(21) Anmeldenummer: **21739276.0**

(22) Anmeldetag: **23.06.2021**

(51) Internationale Patentklassifikation (IPC):
*A61L 9/14* (2006.01)      *A61L 9/20* (2006.01)

(52) Gemeinsame Patentklassifikation (CPC):
**A61L 9/14; A61L 9/20**

(86) Internationale Anmeldenummer:
**PCT/EP2021/067094**

(87) Internationale Veröffentlichungsnummer:
**WO 2021/259976 (30.12.2021 Gazette 2021/52)**

(54) **BEHANDLUNG VON GASEN**

TREATMENT OF GASES

TRAITEMENT DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.06.2020 DE 102020116666**

(43) Veröffentlichungstag der Anmeldung:
**03.05.2023 Patentblatt 2023/18**

(73) Patentinhaber: **Zöllner GmbH**
**89174 Altheim (DE)**

(72) Erfinder: **STEVENS, Prince Charles**
**89174 Altheim (DE)**

(74) Vertreter: **dompatent von Kreisler Selting Werner -
Partnerschaft von Patent- und Rechtsanwälten mbB
Deichmannhaus am Dom
Bahnhofsvorplatz 1
50667 Köln (DE)**

(56) Entgegenhaltungen:
CN-A- 1 692 948       CN-U- 208 809 155
CN-Y- 201 077 544     US-B2- 8 298 482

**EP 4 171 667 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gegenstand der Erfindung ist eine Vorrichtung zur Behandlung von Gasen, insbesondere von Luft und ein entsprechendes Verfahren.

CN 208809155 U bezieht sich auf eine Luftdesinfektionsvorrichtung für Infektionskrankheiten, die einen vertikalen geschlossenen Schrankkörper umfasst und der Schrankkörper mit einer Lufteinlassvorrichtung, einer Oxidations-/Entkeimungsvorrichtung, einer Anionensterilisationsvorrichtung, eine Ultraviolettsterilisationsvorrichtung und einer Auslassvorrichtung von oben nach unten versehen ist. Gemäß dem Gebrauchsmuster wird Stationsluft in die Oxidationsbelüftungssterilisationsvorrichtung zur Belüftung durch die Lufteinlassvorrichtung eingespeist; Wasserstoffperoxid im Wasserstoffperoxidpool kann hauptsächlich Luft oxidieren und desinfizieren; dann strömt die Luft durch das mit dem antibakteriellen Anion im Nanomaßstab beladene Fasernetz, um entkeimt und erneut desinfiziert zu werden. Schließlich tritt Luft in die Ultraviolett-Entkeimungsvorrichtung ein; alle Leitbleche der Ultraviolett-Sterilisationsvorrichtung werden versetzt verteilt, die Strömungsentfernung der Luft wird vergrößert. Während eine Tiefenentkeimung und Desinfektion der desinfizierten Luft unter Bestrahlung einer Ultraviolettlampe durchgeführt wird, wird durch eine Abluftvorrichtung in eine Station abgesaugt, das Recycling wird erreicht. Der Desinfektionseffekt ist sehr gut, die Luft ist leicht in der Station aufzubauen und die Bedienung ist bequem.

CN 107433078 A betrifft eine Luftreinigungsvorrichtung, die ein großes Faserfiltrationssieb, ein Aktivkohlefiltrationssieb und einen Ventilator umfasst, wobei das Aktivkohlefiltrationssieb mit dem Ventilator verbunden ist, wobei die Luftreinigungsvorrichtung ferner einen Nassfilter mit einer porösen Struktur umfasst und positioniert ist zwischen dem großen Faserfiltrationssieb und dem Aktivkohlefiltrationssieb, einem aktiven Sauerstoffsubstanzgenerator, der an der stromaufwärtigen Seite des Nassfilters positioniert ist, und einer ionischen Flüssigkeit. Der aktive Sauerstoffsubstanzgenerator wird zur Erzeugung von aktiven Sauerstoffsubstanzen verwendet. Die Luftreinigungsvorrichtung kann die Aktivität der aktiven Sauerstoffsubstanz in der ionischen Flüssigkeit für eine lange Zeit aufrechterhalten, um die Reinigungseffizienz zu verbessern.

KR 101708799 B1 eine Entkeimungs- und Reinigungsvorrichtung unter Verwendung von Hydroxylradikalen, bestehend aus: einem Gehäuse mit einer Raumeinheit; einer Patrone, die an einer Unterseite einer Raumeinheit des Gehäuses ausgebildet ist und eine Wasserstoffperoxidlösung aufnimmt; und einen Ultraschallwellenvibrator, der die Wasserstoffperoxidlösung in ein Wasserstoffperoxid-Aerosol versetzt und auf einer Oberseite der Raumeinheit des Gehäuses verteilt. Eine Flüssigphasen-Wasserstoffperoxidlösung wird unter Verwendung des Ultraschallwellenvibrators in ein Aerosol mit Tröpfchen von Mikrogröße überführt und mit Ultraschallwellen bestrahlt, so dass eine Kontaktfläche einer Wasserstoffperoxidlösung und Ultraschallwellen vergrößert wird. Die Effizienz der Hydroxylradikale zur Sterilisation und Reinigung von Luft kann erhöht werden.

CN 104501338 A offenbart einen Luftfilter, der eine Hülle, einen Filterkern und einen Ventilator umfasst. Der Filterkern ist in der Schale montiert; ein Lufteinlass ist auf der rechten Seite der Schale ausgebildet; ein Lufteinlass ist auf der linken Seite der Schale ausgebildet; der Lüfter ist in der Schale montiert und auf der linken Seite der Schale positioniert. Ein Lufteinlass, der einen Filterkern umfasst folgt nacheinander einem Filtersieb mit geringem Wirkungsgrad, einer elektrostatischen Staubkammer, einem Wasserstoffperoxid-Reinigungssystem und einem Nano-Aktivkohle-Filtersieb von links nach rechts. Das Wasserstoffperoxid-Reinigungssystem umfasst ein Antioxidationsfilter-Textilmikrofasersieb, das mit Wasserstoffperoxid getränkt ist. Das soll den Vorteil haben, dass mit Hilfe des Wasserstoffperoxids Bakterien und Keime wirksam entfernt werden können und Sekundärverschmutzung zu vermieden. Für die Luft können Sauerstoffquellen und Feuchtigkeit bereitgestellt werden, so dass die Luft dynamischer ist und das Nano-Aktivkohlefiltersieb den Geruch des Wasserstoffperoxids effektiv absorbieren.

KR 20090081930 A stellt eine OH-Radikalerzeugungsvorrichtung bereit, um Verunreinigungen wie flüchtige organische Verbindungen und dergleichen in der Luft wirksam zu oxidieren, zu zersetzen und zu entkeimen, indem eine Reflektatfolie, Hochglanz (ALU-beschichtet) auf eine Innenfläche eines Gehäuses aufgetragen und Licht in einem Wellenlängenbereich angeordnet wird, der bei der Photoreaktion in Längsrichtung in der Mitte des Gehäuses verwendet wird. Eine OH-Radikalerzeugungsvorrichtung umfasst: ein Gehäuse, das ein äußeres Erscheinungsbild des OH-Radikalgenerators bildet mit einem Einlass bzw. einem Auslass, die an dessen Vorder- und Rückseite ausgebildet sind und von denen eine innere Umfangsfläche mit einer Folien -Beschichtungsschicht, einem Wasserstoffperoxidgenerator mit einer Düse, die so angeordnet ist, dass sie mit dem Einlass in Verbindung steht und durch das Gehäuse läuft; und einer UV-Röhre , die von einem Träger in dem Gehäuse schwimmt und Wasserstoffperoxid verdampft, das in den Einlass des Wasserstoffperoxidgenerators fließt, wodurch OH-Radikale zwischen der UV-Röhre und der Folien -Beschichtung erzeugt werden, um sich zu zersetzen und zu oxidieren und in das Gehäuse gesaugte Luft entkeimen und die entkeimte Luft durch den Auslass nach außen ablässt. Es gibt eine Mischzone, die auf eine Länge ausgebildet ist, die nicht kleiner als ein Abstand zwischen den Innenwänden des Gehäuses zwischen einem Einlassende des Gehäuses und einem vorderen Ende der UV-Röhre.

CN 100393361 C betrifft eine an der Wand hängende Luftdekontaminationsvorrichtung zur Vermeidung von Infektionen der Atemwege. Der Lufteinlass am Gehäuse ist mit Lufteinlassjalousien ausgestattet. Der obere Teil der

**EP 4 171 667 B1**

Schale ist mit einem Luftauslassrohr ausgestattet. Die linke Innenseite der Schale ist mit einem Motor mit einstellbarer Drehzahl ausgestattet. Die Antriebswelle ist über einen Riemen mit einem Motor mit einstellbarer Drehzahl verbunden. Der Raum für eine antiseptische Lösung auf der rechten Innenseite der Schale ist mit einer antiseptischen Lösung, einer Führungswelle und einer angetriebenen Welle ausgestattet. Die Antriebswelle, die angetriebene Welle und die Führungswelle sind mit einem Entkeimungsband ausgestattet. Die rechte Seite der Schale ist mit einem Lösungsinjektionsventil und einer Niveauregulierung ausgestattet. Der untere Teil der rechten Seite ist mit einem Lösungsauslassventil ausgestattet. Die innere rechte Seite ist mit Strom für UV-Lampen ausgestattet. Lufteinlassjalousien sind mit einem Photokatalyse-Netz und einem elektrothermischen Quarzrohr ausgestattet. Das Photokatalyse-Netz ist mit einer UV-Röhre ausgestattet. Ein Luftaustritts-Photokatalyse-Netz ist in einer Hülle ausgestattet. Der Luftauslass ist mit einer Aktivkohlefilterdecke und einem Axialventilator ausgestattet. Es kann zur Entkeimung der Station und des Operationssaals, Laboren, öffentlichen Gebäuden und Verkehrsmittel, Wartezimmer, sowie Tagungsräume verwendet werden.

US 6,969,486 B1 beschreibt eine Vorrichtung zum Behandeln von Schadstoffen in einem Gas, insbesondere Luft und umfasst eine Wasserstoffperoxidquelle und einen Behandlungsinjektor zum Erzeugen und Injizieren von dissoziiertem Wasserstoffperoxid in den Gas-Luftstrom. Der Behandlungsinjektor kann ferner ein Injektorgehäuse mit einem Einlass, einem Auslass und einem dazwischen liegenden hohlen Innenraum umfassen. Der Einlass kann in Fluidverbindung mit der Wasserstoffperoxidquelle verbunden sein, so dass Wasserstoffperoxid durch das hohle Innere und in Richtung des Auslasses fließt. Mindestens eine UV-Röhre kann innerhalb des hohlen Innenraums des Injektorgehäuses positioniert sein. Die mindestens eine UV-Röhre kann das durch die Röhre fließende Wasserstoffperoxid katalysieren. Das katalysierte Wasserstoffperoxid kann zur Behandlung von Schadstoffen wie Stickoxiden aus dem Auslass in den Gasstrom injiziert werden.

US 2007/0217944 A1 umfasst ein Verfahren und eine Vorrichtung zum Neutralisieren von Krankheitserregern und chemischen Toxinen in der Luft, in belüfteter Luft sowie in Heizungs- oder Klimaanlagen. Das Pathogen-chemische Toxin-Neutralisationssystem ist gegen ein breites Spektrum von Pathogenen und Toxinen wirksam, enthält handelsübliche Komponenten und kann problemlos in kommerzielle HLK-Systeme integriert werden, in denen große Mengen belüfteter Luft in Echtzeit ohne chemische Reagenzien dekontaminiert werden. Das System verfügt über eine Durchflussreaktionskammer, die mindestens eine UV-Lichtquelle enthält, die kurze intensive Blitze von UV-Energie mit breitem Spektrum emittiert, eine Quelle für wässriges Wasserstoffperoxid, die ein Reservoir oder ein Wasserstoffperoxidgenerator sein kann, und gegebenenfalls eine Ozonquelle. Die Wechselwirkung von UV-Licht und Wasserstoffperoxid erzeugt Hydroxylradikale, die Krankheitserreger und chemische Toxine neutralisieren, wenn sie in Echtzeit durch die Reaktionskammer gelangen. Zu den Krankheitserregern, die durch dieses System neutralisiert werden können, gehören Bakterien, Viren, Sporen, Pilze und Parasiten. In US 2007/0217944 A1 wird Wasserstoffperoxid in einen Bestrahlungsraum geführt, in dem mit "kurzen intensiven Blitzen" entsprechende Hydroxyl-Radikale gebildet werden. Die Interaktion von UV-Licht und Hydroxyl-Radikalen soll die pathogenen und chemischen Gifte neutralisieren. Unter [0042] und [0043] wird festgelegt, dass die wässrige Peroxid-Lösung als versprühte Tröpfchen, als feiner Nebel oder als Dampf durch die Düse bereitgestellt wird. Unter [0050] wird erwähnt, dass das $H_2O_2$ durch eine elektrische Entladung unter Wasser erzeugt wird. In [0053] werden Möglichkeiten erwähnt, Ozon wieder aus der Luft zu entfernen, selbiges wird in [0070] nochmals genauer erläutert. In [0079] wird eine Peroxid-Konzentration von 15% angegeben. Die poröse Membran 107 soll gemäß [0045] dazu dienen, um zusätzliche Oberflächen zu bilden, auf der die freien Radikale mit den Pathogenen reagieren sollen. Es ist jedoch anzunehmen, dass auf einer solchen Oberfläche die Radikale direkt zerstört werden und somit keine Reaktionsfähigkeit entfalten, zumal die Hydroxylradikale bekanntermaßen nur eine Halbwertszeit von max. 1 ns haben, was wiederum für eine Entkeimung bzw. Deaktivierung von Mikroorganismen nachvollziehbarerweise sicherlich allein nicht ausreicht.

Auch in US 6969486 B1 wird die Radikalbildung von Wasserstoffperoxid durch UV-Strahlung verwendet, sodass diese Patentschrift der Vorgenannten nichts Wesentliches hinzufügt. Im Abstract wird bereits beschrieben, dass erst Hydroxyl-Radikale erzeugt werden, die dann dem zu reinigenden Gas beigefügt werden. Hier ist von einem "scrubber" (Wäscher) die Rede. Diese Schrift bezieht sich explizit auf irgendeine eine $H_2O_2$ Quelle und einen Behandlungsinjektor zum Erzeugen und Injizieren von "dissoziiertem" $H_2O_2$ in den Gas-Luftstrom.

WO 2015/059336 A1 betrifft eine Vorrichtung zum Reinigen von Luft durch Emission von Hydroxylradikalen, umfassend: einen Körper, der mit einem ersten Gehäuse für eine erste austauschbare Patrone versehen ist. Die erste austauschbare Patrone und Mittel zum Verursachen der Emanation des Reaktanten von der ersten Patrone sind dadurch gekennzeichnet, dass die erste Patrone einen Reaktanten enthält, der durch Reaktion mit UV-Energie Hydroxylradikale erzeugen kann, und dass die Vorrichtung einen UV-Strahlengenerator umfasst.

Die technische Erzeugung von UV-Energie ist grundsätzlich auf verschiedene Arten möglich. Als effizienteste Methode hat sich heute die Erzeugung über eine Quecksilberdampfentladung durchgesetzt. Bei diesem Prozess wird Quecksilber (Hg) in einem mit Edelgas gefüllten Reaktionsraum mittels aktiver Wärmezufuhr verdampft. In dieser quecksilberdampfgesättigten Edelgasatmosphäre werden nun durch Zuführung elektrischer Energie die Elektronen der Quecksilberatome angeregt. Fallen diese hernach auf ihr stabiles Energieniveau zurück, so geben sie die zuvor zugeführte Energie primär in Form von UV-Energie wieder ab.

3

Das Strahlungsniveau, respektive die messbare spektrale Energieverteilung, welche von dem Plasma emittiert wird, ist dabei in erster Linie abhängig von der Menge der freien Quecksilberatome, dem Gasdruckverhältnis und dem Niveau der zugeführten Energie.

UV-C-Energie (vor allem die bei niedrigem Dampfdruck, mit hoher Ausbeute (30 bis 40 % der angelegten elektrischen Leistung) anregbare Emissionslinie des Quecksilberdampfs bei 253,652 nm) findet in der physikalischen Entkeimungstechnik eine technische Anwendung (siehe auch Quecksilberdampflampen). Während bei 280 nm (Absorptionsmaximum der meisten Proteine) die darin eingebaute Aminosäure Tryptophan die ultraviolette Strahlung absorbiert, werden bei 265 nm Nukleinsäuren am stärksten geschädigt. Bei etwa 245 nm absorbieren vor allem die Nukleinsäuren, während Proteine hier ein relatives Absorptionsminimum zwischen dem Absorptionsmaximum um 280 nm durch aromatische Aminosäuren (Tryptophan, Tyrosin und Phenylalanin) und der Absorption durch die Peptidbindung zwischen den einzelnen Aminosäuren (Maximum bei etwa 220 nm) zeigen. Daher ist bei 254 nm (Primärstrahlung der Niederdruck-Quecksilberdampfentladung) auch die Bestrahlung von Proteinlösungen (etwa Tierseren für die Zellkultur) zur Inaktivierung darin enthaltener Viren und Bakterien möglich.

Für nahezu alle Mikroorganismen ist die tödliche Dosis an UV-C Energie bekannt, ab der die Zelle die Reproduktionseigenschaft verliert, inaktiviert wird und keine Selbstreparatur-Mechanismen aktiviert werden können. Aufgrund der Zellstruktur ist die Letaldosis unterschiedlich hoch. Pathogene Keime und Hefen sind äußerst empfindlich gegenüber UV-C. Manche Schimmelpilzsporen benötigen eine höhere UV-Dosis. Dies ist erforderlichenfalls bei der Auslegung zu berücksichtigen.

Am Ende der Strahlerlebensdauer muss eine ausreichend hohe UV-Dosis vorhanden sein, um in der definierten Bestrahlungszeit eine entsprechende Entkeimungsleistung sicherzustellen durch einen eingebauten Betriebsstundenzähler. Die Letaldosis ist je nach Mikroorganismus und Zellstruktur / Zelltyp unterschiedlich hoch. Pathogene Keime sind sehr empfindlich gegenüber UV-C und werden sehr schnell inaktiviert. Schimmelpilzsporen brauchen eine höhere UV-Dosis. In der Praxis wird beispielsweise bei Siegelfolien für Joghurtbecher innerhalb von 2 Sekunden eine Keimreduktion von 99,5 % bis 99,9 % erzielt.

Jede UV-Strahlenquelle hat eine elektrische Leistungsaufnahme, von der ein Teil im UV-C Bereich emittiert wird. Die im UV-C Bereich emittierte Strahlung wird zu 90 % bei 254 nm erzeugt, der für die Entkeimung wirksamen Wellenlänge. Diese wird als Strahlungsdosis bezeichnet. Die Strahlungsdosis ist also die Energie, die pro Zeiteinheit vom Strahler abgestrahlt wird.

Die Bestrahlungsstärke oder auch Intensität genannt ($\mu$W oder mW) ist die pro Flächeneinheit (cm$^2$) auftreffende Strahlungsdosis. Für ihre Höhe entscheidend ist die optimale Ausnutzung des im Gerät eingebauten Strahlers sowie der Abstand zur zu entkeimenden Sache. Multipliziert man die Bestrahlungsstärke/Dosis mit der Zeit (s), so ergibt sich die UV-Dosis / Bestrahlungsdosis:

$$\text{UV-Dosis (mWs/cm}^2\text{)} = \text{Intensität (mW/cm}^2\text{)} \times \text{Zeit (s)}$$

Je höher die Strahlungsleistung und je länger die Bestrahlungszeit, desto größer die desinfizierende Wirkung. Populär gesagt: Bakterien, Viren, Hefe- und Schimmelpilze haben keine Chance gegen Ultraviolett. Denn zusätzliche Resistenz kann nach wissenschaftlichen Erkenntnissen nicht erworben werden. Die meisten pathogenen Keime sind gegenüber UV-Strahlen sogar besonders empfindlich. Ein wichtiger Vorzug der physikalischen Desinfektion, da diese zum Beispiel auch dann funktioniert, wenn Keime bereits eine Resistenzbildung gegen konventionelle Desinfektionsmaßnahmen (Alkohol, Antibiotika und dergleichen) erworben haben. An dieser Stelle sei auf die MRSA (Multi-resistenter Staphylococcus aureus) Problematik hingewiesen, mit denen viele medizinische Einrichtungen an die Grenze der bisher praktizierten Entkeimung und Prävention angekommen sind. Dieser Umstand der physikalischen Entkeimung funktioniert bei allen Mikroorganismen, egal ob es dabei um häufig auftretende E.Coli Bakterien, Fäkalkeime, TBC, SARS, Anthrax oder Legionellen geht. Eine ausreichende UV-Dosis ist allerdings wesentliche Grundlage - und setzt eine entsprechende Geräteentwicklung voraus.

Der Fokus der vorliegenden Erfindung liegt also insbesondere auf den folgenden Eigenschaften:

a) Entkeimung von Gasen, insbesondere Luft

b) Filtrierung und Eliminierung von Pigmentschmutz, Staub, sowie mikrobiologischen Pathogenen

Die vorgenannte Aufgabe der vorliegenden Erfindung wird in einer ersten Ausführungsform gelöst durch eine Vorrichtung (1) zur Behandlung von Gasen mit

a) einem Gaseinlass (2) für einen Strom des zu behandelnden Gases,

b) einem mit Wasserstoffperoxid und/ oder Persäuren oberflächlich beladenen gasdurchlässigen festen Träger (3), wobei der Träger (3) eine Keramik, ein Gewebe, Gestrick, Gewirke, Gelege, Vlies und/ oder Vlies umfasst,

c) einer Strömungsmaschine (4) zur Beförderung des Gasstroms vom Gaseinlass (2) über den Träger (3) in

d) einen Bestrahlungsraum (5) mit einer UV-C-Lichtquelle (6).

Der Begriff Gas bezeichnet im Rahmen der Erfindung jedwedes Gas oder Gasgemisch, insbesondere Luft, aber auch Abluft von Geräten oder Apparaten.

Der wesentliche Kern der vorliegenden Erfindung besteht darin, dass Wasserstoffperoxid und/ oder Persäuren auf einen festen Träger aufgebracht wird und somit oxidativ und nicht über die entsprechenden Radikale wirkt.

Insbesondere wird erfindungsgemäß ein Nassfilter (Träger 3) mit Peroxid oberflächlich beladen, das von der durchströmenden Luft aufgenommen wird.

Die erfindungsgemäße Vorrichtung und das Verfahren können nicht nur mit Wasserstoffperoxid betrieben werden, sondern vorzugsweise mit einer speziellen Reinigerlösung enthaltend beispielsweise 3 Gew.% -iger Peressigsäure, die speziell bei Raumtemperatur einen wesentlich besseren Wirkungsgrad hat als herkömmliches Wasserstoffperoxid. Ebenso erlaubt es die Erfindung mit Ethanol und/oder Propanol als Lösungsmittel für das Peroxid zu arbeiten, und deren Mischungen untereinander.

Erfindungsgemäß werden sowohl Viren und Bakterien inaktiviert als auch Gerüche und Lösemitteldämpfe zu neutralisiert.

Vorzugsweise ist das System selbstregulierend, und bedarf keiner besonderen Einstellungen des Betreibers. Der mit $H_2O_2$ und/oder Alkohol beladene Träger (3) (Nassfilter) ist in der Lage schon beim ersten Eintritt des Luft/-Gasstroms vorhandene Mikroorganismen, Bakterien und Keime zu inaktivieren. Die im Stand der Technik erwähnten UV-Blitze sind sicherlich nicht zielführend, daher arbeiten wir mit permanenter UV-C Bestrahlung.

Erfindungsgemäß liegt der Fokus auf der Filtrierung und Eliminierung von Pigmentschmutz, Staub, sowie mikrobiologischen Pathogenen.

Eine weitere bevorzugte Ausführungsform der Erfindung ist dadurch gekennzeichnet, dass die Strömungsmaschine (4) im Bereich des Bestrahlungsraums (5), insbesondere im Bereich der UV-C Lampen (6), besonders bevorzugt jedoch im Bereich des Ausgangs des Bestrahlungsraums (5) angeordnet ist. Diese Anordnung der Bauteile hat den Vorteil, dass die Oberfläche der Strömungsmaschine (4) nur mit dem behandelten Gasstrom (2) bei einer geringen Reinigungsmittelkonzentration und geringer sonstiger Belastung des Gasstroms in Kontakt kommt.

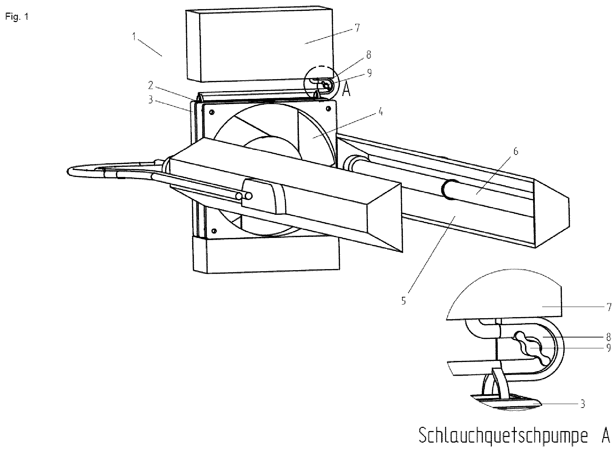

In der Fig. 1 wird eine Ansicht einer entsprechenden Vorrichtung (1) dargestellt. Diese besteht im Wesentlichen aus einem festen Träger (3), dessen, insbesondere innere, Oberfläche mit Wasserstoffperoxid, insbesondere einer wässrigen Lösung, belegt ist. Alternativ kann der Träger (3) auch eine oberflächliche Belegung mit einer alkoholischen Wasserstoffperoxidlösung enthalten. Beide Alternativen weisen ein gewisse biozide, bakterizide und/ oder viruzide Wirkung auf, die bereits für sich geeignet ist, das zu behandelnde Gas, insbesondere Luft zu reinigen.

Der Gasstrom wird mittels einer Strömungsmaschine (4), beispielsweise eines Ventilators, über die Oberfläche des Trägers (3) geführt. Die Anordnung der Reihenfolge des Trägers (3) und der Strömungsmaschine (4) ist dabei frei wählbar. Wird der Gasstrom zunächst durch die Strömungsmaschine (4) und dann über den Träger (3) geführt, so kommt die Strömungsmaschine (4), beispielsweise ein Ventilator, nicht mit dem Wasserstoffperoxid in Kontakt. Dadurch kann die Auswahl der Oberflächenbeschaffenheit der Strömungsmaschine (4) vereinfacht werden.

Das Material des Trägers (3) bzw. dessen Oberfläche kann vielfältig ausgestaltet sein. Voraussetzung ist naturgemäß eine gewisse Stabilität gegenüber Wasserstoffperoxid bzw. dem entsprechenden Lösungsmittel. So umfasst die Struktur des Trägers (3) erfindungsgemäß eine Keramik, ein Gewebe, Gestrick, Gewirke, Gelege, Vlies und/ oder Vlies.

Anstelle von Wasserstoffperoxid oder auch zusätzlich dazu können im Sinne der vorliegenden Erfindung auch Persäuren eingesetzt werden, um den Träger zu beladen. Im Sinne der vorliegenden Erfindung umfassen Persäuren beispielsweise Peressigsäure, Peroktansäure, Pernonansäure und ε-Phthalamid-Peroxo-Capronsäure. Auch deren Gemische untereinander sowie mit Wasserstoffperoxid können erfindungsgemäß eingesetzt werden. In einer bevorzugten Ausführungsform ist der Träger (3) mit Wasserstoffperoxid und/oder Persäuren in einem Lösungsmittel beladen. Bevorzugt umfasst das Lösungsmittel Wasser und/oder Alkohol, insbesondere Ethanol und/oder i-Propanol.

In der Fig. 1 ist die Verbindung des Trägers (3) mit dem Vorratsbehälter (7) dargestellt. Letzterer kann mit Wasserstoffperoxid bzw. einer wässrigen Lösung davon befüllt sein. Durch eine Verbindungsleitung (8) kann des Wasserstoffperoxid von dem Vorratsbehälter (7) kann das Wasserstoffperoxid und/ oder die Persäuren auf die Oberfläche des Trägers (3) gelangen, der sich beispielsweise in einem offenen Gefäß befindet. Die Dosierung der Menge an Wasserstoffperoxid und/ oder der Persäuren ist in einfacher Weise durch eine Schlauchpumpe oder eine sonstige Dosiereinrichtung möglich.

In Fig. 1 ist in der vergrößerten Darstellung der Vorratsbehälter (7), der hier über die Verbindungsleitung 8 zur Benetzung der Oberfläche des Trägers (3) verbunden ist. Die Größe des Vorratsbehälters (7) richtet sich nach dem gewünschten Anwendungszweck und insbesondere dem Volumen des Gasstroms.

Vorzugsweise umfasst die Verbindungsleitung (8) zwischen dem Vorratsbehälter (7) und dem Träger (3) eine Dosiereinrichtung (9) zur Regulierung der Durchflussmenge des Wasserstoffperoxids und/ oder der Persäuren bzw. der entsprechenden Lösung. Besonders bevorzugt geeignet ist hierfür eine Schlauchquetschpumpe.

Der Gasstrom durchströmt den Träger (3) und kommt hierbei insbesondere mit dem Wasserstoffperoxid und/ oder den Persäuren beziehungsweise der entsprechenden Lösung in Kontakt. Hierbei hat sich gezeigt, dass das Wasserstoffperoxid beziehungsweise die entsprechende Lösung als Oxidationsmittel für die Kontamination wirken und so eine Reinigung des Gasstroms 2 bewirken können. Hydroxylradikale können sich hier praktisch nicht bilden. Als einfachste Strömungsmaschine (4) für den Gasstrom eignet sich ein Ventilator bzw. ein Verdichter. Beide Begriffe werden hier synonym gebraucht.

Der Gasstrom wird im weiteren Verlauf dann in einen Bestrahlungsbereich (5) geführt, der mit wenigstens einer UV-Röhre (6) bestückt ist.

Durch die erfindungsgemäße Bestrahlung des peroxidhaltigen Gasstroms mit UV-C-Strahlung entstehen OH-Radikale. Die gebildeten OH-Radikale verbessern die Gasreinigung. Zudem ermöglicht die Bestrahlung die Ausnutzung des Wasserstoffperoxids zu verbessern, indem nicht verbrauchtes $H_2O_2$ zu OH-Radikalen umgesetzt wird. Dadurch wird die Desinfektionswirkung des Peroxids verbessert.

Die UV-Röhre (6) ist insbesondere dazu geeignet, kontinuierlich UV-C-Strahlung abzugeben. Neben der gegebenenfalls stattfindenden Bildung von Hydroxyl-Radikalen hat die UV-C-Strahlung auch eine direkte Desinfektionswirkung auf den Gasstrom im Sinne einer physikalischen Desinfektion.

Unter UV-Strahlung wird hier, in Übereinstimmung mit der Definition der Weltgesundheitsorganisation (WHO), elektromagnetische Strahlung mit einer Wellenlänge im Bereich von 1 nm bis 400 nm, insbesondere 100 bis 400 nm, verstanden. Das Spektrum der UV-Strahlen wird weiter untergliedert in UVA- (315 nm bis 380 nm), UVB- (280 nm bis 315 nm) und UVC-Strahlung (100 nm bis 280 nm). Das UV-Spektrum unterhalb von 100 nm wird als extremes UV bezeichnet. Unterhalb einer Wellenlänge von 0,25 nm beginnt das Spektrum der Röntgenstrahlung. Die UV-Strahlungsquelle umfasst in einer bevorzugten Ausführungsform eine Quecksilberdampfröhre und/oder eine oder mehrere UVC-LEDs, insbesondere in Form einer Bandanbringung. Der Vorteil von UVC-LEDs ist neben einer sehr hohen Lebensdauer die sehr kleine Bauteilgröße sowie eine einfache und verbrauchsarme Elektronik, die einen unkomplizierten Gerätebau ermöglichen. Grundsätzlich kann allerdings jede im Stand der Technik bekannte UV-Strahlungsquelle zum Einsatz kommen. Vorzugsweise ist die UV-Strahlungsquelle unmittelbar stromaufwärts des letzten Bauteils des Trägers (3) oder der Strömungsmaschine (4) angebracht.

Die Wellenlänge der von der UV-Strahlungsquelle emittierten UV-Strahlung liegt vorzugsweise in einem Bereich von 240 bis 260 nm, besonders bevorzugt 254 bis 260 nm, insbesondere 254 bis 258 nm. Die Strahlungsdosis liegt vorzugsweise in einem Bereich von 200 nm bis 300 nm, besonders bevorzugt in einem Bereich von 254 nm bis 260 nm, insbesondere 254 nm bis 258 nm. Vorzugsweise werden Strahlungsquellen mit einer langen Haltbarkeit eingesetzt, insbesondere bis zu 12.000 Betriebsstunden bei 18.000 Zündungen. Die UV-Strahlungsquelle kann eine einstellbare Strahlungsintensität aufweisen.

Die UV-Strahlungsquelle ist in einer bevorzugten Ausführungsform mit einer Abschirmung versehen. Auf diese Weise kann die austretende Strahlung gesteuert werden und insbesondere verhindert werden, dass der Benutzer der erfindungsgemäßen Vorrichtung der UV-Strahlung ausgesetzt wird. Die Abschirmung kann insbesondere durch ein Edelstahlblech realisiert werden.

Überraschenderweise hat sich gezeigt, dass Strahlungsanteile im UVC-Bereich eine besonders starke Aktivierung des Gas-, insbesondere Luftstroms bewirken. Diese Wirkung kann noch gesteigert werden, wenn die eingesetzte Strahlung ausschließlich aus UVC-Strahlung besteht. So lässt sich beispielsweise die Wirkung von Wasserstoffperoxid durch UVC-Strahlung um das 4.000-fache steigern. UVC-Strahlung weist zudem den besonderen Vorteil auf, dass sie etwa im Bereich einer Wellenlänge von 250 nm bis 270 nm, insbesondere im Bereich um 258 nm, einen stark keimtötenden bzw. bakteriziden und viruziden Effekt aufweist. Diese Eigenschaft lässt sich darauf zurückführen, dass die kurzwellige und energiereiche UVC-Strahlung durch eine Schädigung der DNS die Aufrechterhaltung des Stoffwechsels und der Zellteilung stoppt, sodass derart geschädigte Zellen letztlich absterben. Dabei ist eine mutationsbedingte Resistenzbildung ausgeschlossen. Diese desinfizierende Wirkung nimmt mit steigender Komplexität der angegriffenen Organismen ab. So lassen sich Viren und Bakterien wesentlich leichter zerstören als beispielsweise Pilze oder Pilzsporen. Da UVC-Strahlen grundsätzlich keine festen Stoffe durchdringen, ist eine sichere Abschirmung jedoch einfach möglich.

Die Strahlungsintensität liegt vorzugsweise in einem Bereich von 100 nm bis 300 nm, besonders bevorzugt in einem Bereich von 240 nm bis 260 nm, insbesondere 254 nm bis 260 nm.

Die Wirksamkeit einer Desinfektionsmethode auf Basis von UV-C Energie steht bekanntermaßen in direktem Zusammenhang mit der angewandten Dosis (= Zeit x eingestrahlte Energie/Fläche). Hohe Intensitäten während einer kurzen Zeit, oder geringe Intensitäten über einen längeren Zeitraum sind praktisch austauschbar und beinahe gleichwertig in der Desinfektionswirkung. Die Dosis wird in $\mu W{*}s/cm^2$ angegeben, häufig auch in $J/m^2$.

**EP 4 171 667 B1**

Die erfindungsgemäße Vorrichtung und das erfindungsgemäße Verfahren können beispielsweise zur Verbesserung der Hygiene im Bereich Krankenhaus und Altenheim, Hotel und Gastronomie, in öffentlichen Einrichtungen, wie insbesondere öffentlichen Toiletten, aber auch im privaten Haushalt, insbesondere bei Tierhaltung, sowie im privaten Bereich bei Allergien, wie beispielsweise gegen Hausstaubmilben, Tierhaare und dergleichen, Einsatz finden. Die Erfindung kann der Qualitätssicherung der Hygiene in Industrie, Pharmazie und Medizin dienen.

Durch die erfindungsgemäße Bestrahlung des Gas-Luftstroms mit UV-C-Energie werden insbesondere organische Bestandteile der Waschflotte zersetzt, sodass freie Radikale entstehen. Diese freien Radikale führen zu einer verbesserten Entkeimungsleistung.

Überraschenderweise hat sich gezeigt, dass sich durch die Bestrahlung mit UV-Energie in Kombination mit einem mit Wasserstoffperoxid beladenem Träger (3) besondere Vorteile ergeben. So macht sich die erfindungsgemäße Vorrichtung (1) zunutze, dass der Gasstrom nur kurze Zeit in dieser verbleibt. Die mittels UV-C-Strahlung gebildeten Radikale haben im Allgemeinen nur eine kurze Lebensdauer. Die Bildung freier Radikale findet dann unmittelbar in der Vorrichtung (1) statt. Diese Vorgehensweise bietet den besonderen Vorteil, dass die desinfizierende Wirkung der UV-Energie selbst unmittelbar auf den Gas-, insbesondere Luftstrom wirkt.

Überraschenderweise hat sich gezeigt, dass Strahlungsanteile im UVC-Bereich eine besonders starke Aktivierung des Gasstroms bewirken. Diese Wirkung kann noch gesteigert werden, wenn die eingesetzte Strahlung ausschließlich aus UVC-Strahlung besteht. So lässt sich beispielsweise die Wirkung von Wasserstoffperoxid durch UVC-Strahlung um das 4.000-fache steigern. UVC-Strahlung weist zudem den besonderen Vorteil auf, dass sie etwa im Bereich einer Wellenlänge von 250 nm bis 270 nm, insbesondere im Bereich um 258 nm, einen stark keimtötenden bzw. bakteriziden und viruziden Effekt aufweist. Diese Eigenschaft lässt sich darauf zurückführen, dass die kurzwellige und energiereiche UVC-Strahlung durch eine Schädigung der DNS die Aufrechterhaltung des Stoffwechsels und der Zellteilung stoppt, sodass derart geschädigte Zellen oder auch Viren letztlich absterben. Dabei ist eine mutationsbedingte Resistenzbildung ausgeschlossen. Diese desinfizierende Wirkung nimmt mit steigender Komplexität der angegriffenen Organismen ab. So lassen sich Viren und Bakterien wesentlich leichter zerstören als beispielsweise Pilze oder Pilzsporen. Da UVC-Strahlen grundsätzlich keine festen Stoffe durchdringen, ist eine sichere Abschirmung jedoch einfach möglich.

Eine weitere bevorzugte Ausführungsform der Erfindung ist dadurch gekennzeichnet, dass die Strömungsmaschine (4) im Bereich des Bestrahlungsraums (5), insbesondere im Bereich der UV-Lampen (6), besonders bevorzugt jedoch im Bereich des Ausgangs des Bestrahlungsraums (5) angeordnet ist. Diese Anordnung der Bauteile hat den Vorteil, dass die Oberfläche der Strömungsmaschine (4) nur mit dem behandelten Gasstrom bei einer geringen Peroxidkonzentration und geringer sonstiger Belastung des Gasstroms in Kontakt kommt.

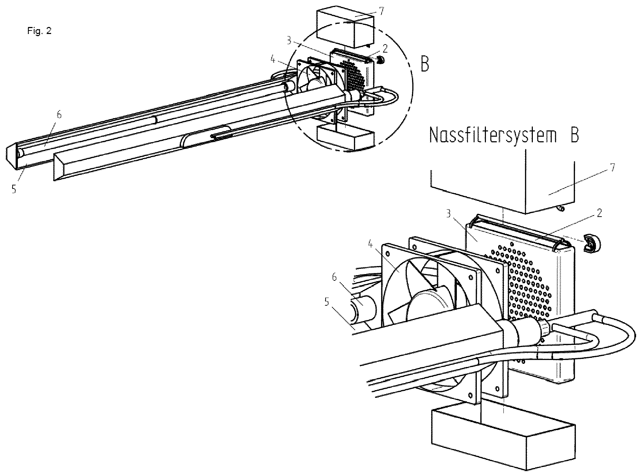

Fig. 2 zeigt eine andere perspektivische Darstellung der Fig. 1 mit einem Detailausschnitt B einer möglichen Anordnung in Strömungsrichtung des Trägers (3), gefolgt von der Strömungsmaschine (4) und dem Bestrahlungsraum (5), der mit den UV-Röhren (6) bestückt ist.

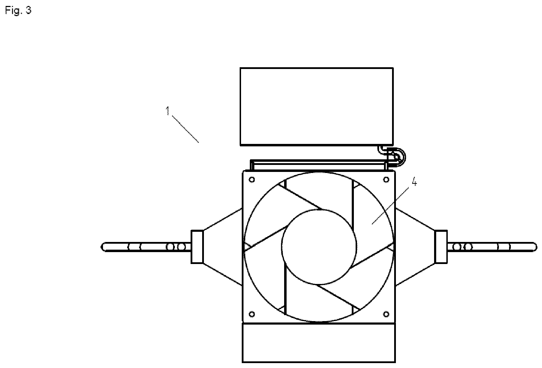

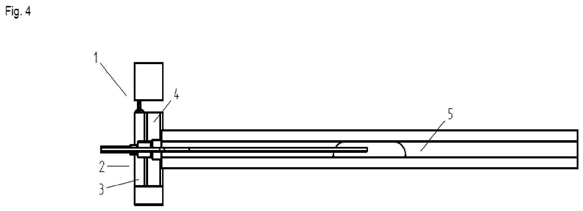

In den Fig. 3 und 4 werden eine Frontansicht bzw. eine Seitenansicht der erfindungsgemäßen Vorrichtung dargestellt.

Eine weitere Ausführungsform der vorliegenden Erfindung besteht darüber hinaus in einem Verfahren zur Behandlung von Gasen, insbesondere Luft mit einer Vorrichtung wie oben beschrieben, das dadurch gekennzeichnet ist, dass man das zu behandelnde Gas über einen mit Wasserstoffperoxid und/oder Persäuren beladenen Träger (3) mit einer Strömungsmaschine (4) in einen Bestrahlungsraum (5) mit einer UV-C-Lichtquelle (6) leitet. Durch die Konfiguration der Vorrichtung und die oben beschriebene Reihenfolge der Bauteile, nämlich Träger (3), Strömungsmaschine (4) und Bestrahlungsraum (5) wird die Führung des Gas-, insbesondere Luftstrom bestimmt.

Vorzugsweise wird die UV-C-Lichtquelle in dem Bestrahlungsraum (5) kontinuierlich betrieben, sodass der Bestrahlungsraum (5) kontinuierlich mit UV-C-Strahlung bestrahlt ist.

In einer bevorzugten Ausführungsform der Erfindung ist im Bereich es Gaseinlasses (2), insbesondere zwischen dem Gaseinlass (2) und dem Träger (3), ein Partikelfilter angeordnet. Ein derartiger Partikelfilter kann dafür sorgen, dass keine festen Verunreinigungen in das System eingetragen werden, die zu einer Verminderung der Entkeimungsbeziehungsweise Reinigungsleistung führen könnten. Sofern partikelhaltige Verschmutzungen mit Schwermetallen in das System eindringen, kann eine katalytische Wirkung entstehen. Auf diese Weise kann die Effizienz der Entkeimung beeinträchtigt werden. Überraschenderweise hat sich gezeigt, dass diese Nachteile, die insbesondere auch Luftdesinfektionsvorrichtungen aus dem Stand der Technik betreffen, durch den bevorzugten Einsatz eines Partikelfilters vermieden werden können.

In einer weiteren bevorzugten Ausführungsform weist die erfindungsgemäße Vorrichtung eine bakterienhemmende Innenbeschichtung auf.

Die erfindungsgemäße Vorrichtung kann insbesondere als mobiles System ausgestaltet sein.

Überraschenderweise hat sich gezeigt, dass mit der erfindungsgemäßen Vorrichtung nicht nur Keime, insbesondere Bakterien und Viren, inaktiviert werden können, sondern insbesondere auch Gerüche und Lösungsmitteldämpfe neutralisiert werden können.

Bezugszeichenliste

1    Vorrichtung zur Behandlung von Gasen
2    Gaseinlass
3    Träger
4    Strömungsmaschine
5    Bestrahlungsraum
6    UV-C-Lichtquelle
7    Vorratsbehälter
8    Verbindungsleitung
9    Schlauchquetschpumpe

**Patentansprüche**

1.    Vorrichtung (1) zur Behandlung von Gasen mit

a) einem Gaseinlass (2) für einen Strom des zu behandelnden Gases,
b) einem mit Wasserstoffperoxid und/ oder Persäuren oberflächlich beladenen gasdurchlässigen festen Träger (3), wobei der Träger (3) eine Keramik, ein Gewebe, Gestrick, Gewirke, Gelege, Vlies und/ oder Vlies umfasst,
c) einer Strömungsmaschine (4) zur Beförderung des Gasstroms vom Gaseinlass (2) über den Träger (3) in
d) einen Bestrahlungsraum (5) mit einer UV-C-Lichtquelle (6).

2.    Vorrichtung nach Anspruch 1, **dadurch gekennzeichnet, dass** die Strömungsmaschine (4) einen Ventilator umfasst.

3.    Vorrichtung nach einem der Ansprüche 1 oder 2, **dadurch gekennzeichnet, dass** die Strömungsmaschine (4) im Strömungsverlauf des zu behandelnden Gases vor und/oder hinter dem Träger (3) angeordnet ist.

4.    Vorrichtung nach einem der Ansprüche 1 bis 3, **dadurch gekennzeichnet, dass** die Strömungsmaschine (4) im Bereich des Bestrahlungsraums (5) angeordnet ist.

5.    Vorrichtung nach Anspruch 4, **dadurch gekennzeichnet, dass** die Strömungsmaschine (4) im Bereich des Ausgangs des Bestrahlungsraums (5) angeordnet ist.

6.    Vorrichtung nach einem der Ansprüche 1 bis 5, **dadurch gekennzeichnet, dass** der Träger (3) mit einem Vorratsbehälter (7) über eine Verbindungsleitung (8) zur Benetzung der Oberfläche des Trägers (3) verbunden ist.

7.    Vorrichtung nach Anspruch 6, **dadurch gekennzeichnet, dass** die Verbindungsleitung (8) eine Dosiereinrichtung, insbesondere eine Schlauchquetschpumpe (9) umfasst.

8.    Vorrichtung nach einem der Ansprüche 1 bis 7, **dadurch gekennzeichnet, dass** der Träger (3) mit Persäuren beladen ist, die insbesondere in einem Lösungsmittel gelöst sind.

9.    Vorrichtung nach einem der Ansprüche 1 bis 8, **dadurch gekennzeichnet, dass** diese ein Partikelfilter im Bereich des Gaseinlasses (2) aufweist.

10.   Verfahren zur Behandlung von Gasen mit einer Vorrichtung nach einem der Ansprüche 1 bis 9, **dadurch gekennzeichnet, dass** man das zu behandelnde Gas über einen mit Wasserstoffperoxid und/ oder Persäuren beladenen Träger (3) mit einer Strömungsmaschine (4) in einen Bestrahlungsraum (5) mit einer UV-C-Lichtquelle (6) leitet.

11.   Verfahren nach Anspruch 10, **dadurch gekennzeichnet, dass** man als Gas Luft einsetzt.

**Claims**

1.    A device (1) for treating gases, comprising

a) a gas inlet (2) for a flow of the gas to be treated,
b) a gas-permeable solid support (3) surface-charged with hydrogen peroxide and/or peracids, wherein said support (3) includes a ceramic, a woven fabric, a knitted fabric, a crocheted fabric, a laid web, a non-woven fabric, and/or a fleece,
c) a flowing machine (4) for conveying the gas flow from the gas inlet (2) through the support (3) into
d) an irradiation chamber (5) with a UV-C light source (6).

2. The device according to claim 1, **characterized in that** said flowing machine (4) includes a fan.

3. The device according to either of claims 1 or 2, **characterized in that** said flowing machine (4) is provided upstream and/or downstream of the support (3) in the course of flow of the gas to be treated.

4. The device according to any of claims 1 to 3, **characterized in that** said flowing machine (4) is provided in the area of the irradiation chamber (5).

5. The device according to claim 4, **characterized in that** said flowing machine (4) is provided in the area of the exit of the irradiation chamber (5).

6. The device according to any of claims 1 to 5, **characterized in that** said support (3) is connected to a storage container (7) via a connecting line (8) for wetting the surface of the support (3).

7. The device according to claim 6, **characterized in that** said connecting line (8) comprises a metering means, especially a peristaltic pump (9).

8. The device according to any of claims 1 to 7, **characterized in that** said support (3) is charged with peracids, in particular, with peracids dissolved in a solvent.

9. The device according to any of claims 1 to 8, **characterized by** having a particle filter in the area of the gas inlet (2).

10. A process for treating gases using a device according to any of claims 1 to 9, **characterized in that** the gas to be treated is passed over a support (3) charged with hydrogen peroxide and/or peracids using a flow machine (4) into an irradiation chamber (5) with a UV-C light source (6).

11. The process according to claim 10, **characterized in that** air is employed as said gas.

**Revendications**

1. Dispositif (1) pour traiter des gaz, comprenant

a) une entrée de gaz (2) pour un courant du gaz à traiter,
b) un support solide perméable aux gaz (3) dont la surface est chargée avec du peroxyde d'hydrogène ou des peracides, dans lequel ledit support (3) comprend une céramique, un tissu tissé, tricot, tissu de mailles, natte, tissu non tissé, et/ou nappe,
c) une machine à flux (4) pour transporter ledit flux de gaz à partir de l'entrée de gaz à travers le support (3) dans
d) une chambre d'irradiation (5) avec une source de lumière UV-C (6).

2. Dispositif selon la revendication 1, **caractérisé en ce que** ladite machine à flux (4) comprend un ventilateur.

3. Dispositif selon l'une des revendications 1 ou 2, **caractérisé en ce que** ladite machine à flux (4) est située en amont ou en aval du support (3) dans l'écoulement du flux du gaz à traiter.

4. Dispositif selon l'une quelconque des revendications 1 à 3, **caractérisé en ce que** ladite machine à flux (4) est située dans la région de la chambre d'irradiation (5).

5. Dispositif selon la revendication 4, **caractérisé en ce que** ladite machine à flux (4) est située dans la région de la sortie de la chambre d'irradiation (5).

**6.** Dispositif selon l'une quelconque des revendications 1 à 5, **caractérisé en ce que** ledit support (3) est relié avec un réservoir (7) par une ligne de raccordement (8) pour mouiller la surface du support (3).

**7.** Dispositif selon la revendication 6, **caractérisé en ce que** ladite ligne de raccordement (8) comprend un moyen de dosage, notamment une pompe péristaltique (9).

**8.** Dispositif selon l'une quelconque des revendications 1 à 7, **caractérisé en ce que** ledit support (3) est chargé avec des peracides, notamment des peracides dissouts dans un solvant.

**9.** Dispositif selon l'une quelconque des revendications 1 à 8, **caractérisé en ce qu'**il présente un filtre à particules dans la région de l'entrée de gaz (2).

**10.** Procédé pour traiter des gaz en utilisant un dispositif selon l'une quelconque des revendications 1 à 9, **caractérisé en ce que** le gaz à traiter est passé par un support (3) chargé avec du peroxyde d'hydrogène et/ou des peracides au moyen d'une machine à flux (4) dans une chambre d'irradiation (5) avec une source de lumière UV-C (6).

**11.** Procédé selon la revendication 10, **caractérisé en ce que** de l'air est utilisé comme gaz.

Fig. 1

Schlauchquetschpumpe A

Fig. 2

Nassfiltersystem B

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 208809155 U
- CN 107433078 A
- KR 101708799 B1
- CN 104501338 A
- KR 20090081930 A
- CN 100393361 C
- US 6969486 B1
- US 20070217944 A1
- WO 2015059336 A1